# EUROPEAN PATENT APPLICATION

(11) **EP 4 506 470 A1**
(43) Date of publication of application: **12.02.2025**
(21) Application number: 23784860.1
(22) Date of filing: 21.02.2023
(51) Int. Cl.: C12Q 1/6886, G01N 33/574

(54) **METHOD FOR PREDICTING COLORECTAL CANCER PROGNOSIS USING MRNA OF HSPD1 GENE OR HSP60 PROTEIN ENCODED BY THESE GENES**

(30) Priority: 08.04.2022 KR 20220043843
(71) Applicant: Gil Medical Center, Incheon 21565 (KR)
(72) Inventor: KIM, Jung Ho, Incheon 21565 (KR)
(74) Representative: Office Freylinger
(86) International application number: PCT/KR2023/002486
(87) International publication number: WO 2023/195632

(57) **Abstract**

The present invention relates to: a biomarker for predicting the prognosis of colorectal cancer by using changes in expression level of mRNA of a HSPD1 gene or a HSP60 protein encoded by these genes; and a prognosis prediction method using same. By analyzing the expression level of the HSPD1 gene or the HSP60 protein, the prognoses of colorectal cancer patients can be predicted in clinical practice, and when analysis is carried out in combination with TNM classification, more sophisticated prediction is possible and personalized strategies can be designed.

## Description

### BACKGROUND OF THE INVENTION

### 1. Field of the Invention

The present invention relates to a method for predicting the prognosis of colorectal cancer using mRNA of HSPD1 gene or HSP60 protein encoded by these genes.

### 2. Description of the Related Art

The digestive system is divided into the esophagus, stomach, small intestine, and large intestine, and the large intestine is the last part of the digestive system, where primarily moisture and electrolyte absorption occur. The large intestine is divided into colon, cecum, and rectum, and the colon is further divided into ascending colon, transverse colon, descending colon, and sigmoid colon. Depending on the location of cancer, cancer that occurs in the colon is called colon cancer, and cancer that occurs in the rectum is called rectal cancer, which are collectively called colorectal cancer. The approximate incidence of cancer in each region of the large intestine is known to be 25% in the cecum and ascending colon, 15% in the transverse colon, 5% in the descending colon, 25% in the sigmoid colon, 10% in the rectum-sigmoid colon junction, and 20% in the rectum.

The large intestine is a pipe-shaped tube and is divided into four layers from the inside: the mucosal, submucosal, muscular, and serosal layers. Most colorectal cancers are adenocarcinomas that originate in the mucous membrane of the colon, but others include lymphomas, sarcomas, squamous cell carcinomas, and metastatic lesions from other cancers.

The causes of these colorectal cancers can be largely divided into environmental and genetic factors. Although the cause of colorectal cancer is still unclear, it is believed that environmental factors play a greater role in the development of colorectal cancer than genetic factors, and it is recognized that rapid westernization of diet, especially excessive consumption of animal fats and proteins, is a contributing factor. However, around 5% of colorectal cancers are thought to be caused by a genetic predisposition.

The relationship between diet and colorectal cancer is one of the most studied areas of research, and changes in where people live, such as through immigration, can change the incidence of colorectal cancer based on regional characteristics, regardless of genetic differences. In particular, high calorie intake, excessive consumption of animal fats, high-fat diet, obesity, low fiber intake, weight gain, inflammatory bowel disease, and colorectal polyps are known to increase the risk of colorectal cancer.

Biomarkers, on the other hand, are becoming a major means of advancing personalized medicine, and are divided into nucleic acid-based biomarkers, which are based on DNA and RNA, and protein-based biomarkers, which are based on proteins and their parts. These biomarkers have recently been applied for early diagnosis of various intractable diseases such as cancer, infectious diseases, cardiovascular diseases, stroke, and dementia, as well as for diagnosis of drug response and treatment.

Most patients with cancer, including colorectal cancer, die primarily as a result of cancer metastasis, despite the use of various existing treatment methods (surgery, chemotherapy, and radiotherapy). Most patients whose primary tumor is observed before cancer metastasis have a high possibility of cure, but such patients are difficult to detect, and in most patients, cancer metastasis is already discovered at the time the primary tumor is observed. Most cancer metastases are multiple and systemic, and their presence is difficult to detect, which is why current cancer therapies do not provide satisfactory treatment efficacy. However, cancer metastasis is an inefficient process in which only a small fraction of the cancer cells that make up the primary tumor successfully complete the various stages of the metastatic process and become metastatic cancer. Therefore, by better understanding the metastatic process, discovering clinically and biologically useful therapeutic targets, and developing methods to effectively inhibit them, it will be possible to develop useful treatments that can effectively control death due to cancer metastasis. In addition, if it is possible to predict prognosis early and diagnose high-risk groups for cancer metastasis early, it can be useful for planning future treatment according to prognosis and for personalized treatment for each patient.

As a prior art, Korean Patent Publication No. 1020170089316 discloses a method of determining cancer by reacting the HSP60 protein present in a sample isolated from a patient suspected of having cancer with hydroxylamine and confirming the degradation of the HSP60 protein. Korean Patent No. 101307132 discloses that inhibiting HSP60 has a therapeutic effect on abnormal cell proliferation diseases such as cancer or hyperproliferative vascular disorders by blocking the interaction of HSP60 with the IKK complex, thereby inactivating the NF-kB pathway and inducing apoptosis. Meanwhile, in a non-patent literature paper by Ce'line Hamelin et al. [FEBS Journal 278 (2011) 4845-4859], the expression level of HSP60 protein was measured in the colorectal tissues of normal people and the colorectal tissues of colorectal cancer patients, and it was described that the expression of HSP60 protein significantly increased in the tissues of colorectal cancer patients.

Accordingly, the present inventors identified HSPD1 gene or HSP60 protein with differential expression in the colorectal cancer tissues obtained from colorectal cancer patients and analyzed them in conjunction with survival analysis in order to find biomarkers that can predict the prognosis of colorectal cancer. As a result, it was confirmed that HSPD1 gene or HSP60 protein could be used to predict the prognosis of colorectal cancer patients. In addition, the present invention was completed by confirming that linking the expression of HSPD1 gene or HSP60 protein with TNM stage (tumor, node, and metastasis stage) can more precisely predict the prognosis of colorectal cancer patients.

### SUMMARY OF THE INVENTION

It is an object of the present invention to provide a biomarker for predicting the prognosis of colorectal cancer patients and a method for predicting the prognosis using the biomarker.

To achieve the above object, the present invention provides a composition for predicting the prognosis of a colorectal cancer patient, comprising an agent for measuring the level of mRNA of HSPD1 gene or HSP60 protein encoded by these genes.

The present invention also provides a kit for predicting the prognosis of a colorectal cancer patient, comprising a composition containing an agent for measuring the level of mRNA of HSPD1 gene or HSP60 protein encoded by these genes.

The present invention also provides a method for predicting the prognosis of a colorectal cancer patient comprising the following steps:
(a) a step of measuring the level of mRNA of HSPD1 gene or HSP60 protein encoded by these genes in a sample isolated from a colorectal cancer patient; and
(b) a step of comparing the measured mRNA level or protein level thereof with the mRNA level or protein level thereof of a control sample.

In addition, the present invention provides a method for predicting the prognosis of a colorectal cancer patient comprising the following steps:
(a) a step of measuring the level of mRNA of HSPD1 gene or HSP60 protein encoded by these genes in a sample isolated from a colorectal cancer patient;
(b) a step of comparing the measured mRNA level or protein level thereof with the mRNA level or protein level thereof of a control sample; and
(c) a step of combining and analyzing the information classified according to the TNM stage.

### ADVANTAGEOUS EFFECT

The present invention relates to a biomarker for predicting the prognosis of colorectal cancer by using changes in expression level of mRNA of HSPD1 gene or HSP60 protein encoded by these genes, and a prognosis prediction method using same. By analyzing the expression level of the HSPD1 gene or the HSP60 protein, the prognoses of colorectal cancer patients can be predicted in clinical practice, and when analysis is carried out in combination with TNM classification, more sophisticated prediction is possible and personalized strategies can be designed.

### BRIEF DESCRIPTION OF THE DRAWINGS

Figure 1A is a graph showing the overall survival analysis according to the expression level of HSPD1 in the TCGA COAD data set.
Figure 1B is a diagram showing the hazard ratio analysis of Figure 1A.
Figure 1C is a graph showing the relapse free survival analysis according to the expression level of HSPD1 in the TCGA COAD data set.
Figure 1D is a diagram showing the hazard ratio analysis for Figure 1C.
Figure 2A is a graph showing the results of the overall survival analysis combining the expression level of HSPD1 and the TNM classification in the TCGA COAD data set.
Figure 2B is a diagram showing the results of the overall survival analysis combining the expression level of HSPD1 and the TNM classification in the TCGA COAD data set.
Figure 2C is a diagram showing the overall survival analysis according to the expression level of HSPD1 in the TCGA COAD data set at the late TNM stages (stages 3 and 4).
Figure 3A is a graph showing the relapse free survival analysis combining the expression level of HSPD1 and the TNM classification in the TCGA COAD data set.
Figure 3B is a diagram showing the relapse free survival analysis combining the expression level of HSPD1 and the TNM classification in the TCGA COAD data set.
Figure 3C is a diagram showing the relapse free survival analysis according to the expression level of HSPD1 in the TCGA COAD data set at the late TNM stages (stages 3 and 4).
Figure 4 is a set of photographs showing the results of immunochemical staining for HSP60 in tumor cells (A: unstained normal colon epithelial cells, B: unstained tumor cells, C: tumor cells showing weak cytoplasmic staining, D: tumor cells showing moderate cytoplasmic staining, E: tumor cells showing strong cytoplasmic staining) (magnification: 400 x).
Figure 5A is a graph showing the event free survival analysis according to the HSP60 protein expression level in the GMC (Gachon University Gil Medical Center) colorectal cancer cohort.
Figure 5B is a diagram showing the hazard ratio analysis of Figure 5A.
Figure 5C is a graph showing the disease specific survival analysis according to the HSP60 protein expression level in the GMC (Gachon University Gil Medical Center) colorectal cancer cohort.
Figure 5D is a diagram showing the hazard ratio analysis of Figure 5C.
Figure 6A is a graph showing the event free survival analysis by combining the HSP60 protein expression level and TNM stage.
Figure 6B is a diagram showing the event free survival analysis by combining the HSP60 protein expression level and TNM stage.
Figure 6C is a diagram showing the event free survival analysis according to the expression level of HSPD1 in the GMC colorectal cancer cohort at the late TNM stages (stages 3 and 4).
Figure 7A is a graph showing the disease specific survival analysis by combining the HSP60 protein expression level and TNM stage in the GMC colorectal cancer cohort.
Figure 7B is a diagram showing the disease specific survival analysis by combining the HSP60 protein expression level and TNM stage in the GMC colorectal cancer cohort.
Figure 7C is a diagram showing the disease specific survival analysis according to the expression level of HSP60 in the GMC colorectal cancer cohort at the late TNM stages (stages 3 and 4).

### DESCRIPTION OF THE PREFERRED EMBODIMENTS

Hereinafter, the present invention is described in detail.

The present invention provides a composition for predicting the prognosis of a colorectal cancer patient, comprising an agent for measuring the level of mRNA of HSPD1 gene or HSP60 protein encoded by these genes.

The above HSPD1 (Heat Shock Protein Family D (Hsp60) Member 1) encodes a protein commonly known as HSP60, also known as Cpn60. This protein is a mitochondrial protein and is a type of heat shock protein that responds to high temperatures (heat) to inhibit cellular damage. The heat shock protein acts as a chaperone, binding to proteins that do not exhibit a complete stereostructure to prevent intermolecular association formation and promote regeneration. The heat shock protein is also known to bind to immature proteins shortly after synthesis and play important roles in their higher order structure formation, intracellular transport, membrane permeation of organelles, and covalently bind to denatured proteins to mediate their degradation by proteasome.

The agent for measuring the level of mRNA may include a primer or a probe, but not always limited thereto.

The above primer is a nucleic acid sequence having a short free 3' hydroxyl group that can form complementary base pairs with a template and functions as a starting point for replicating the template strand. The primers can initiate DNA synthesis in the presence of the reagents for the polymerization reaction (i.e., DNA polymerase or reverse transcriptase) and the different four nucleoside triphosphates in an appropriate buffer and at a proper temperature.

The probe may be a probe capable of binding complementarily to a target gene, and the nucleotide sequence of the probe is not limited as long as it is capable of binding complementarily to each gene.

The method for measuring the mRNA expression level includes RT-PCR (reverse transcription polymerase chain reaction), competitive RT-PCR, real-time RT-PCR, RNase protection assay (RPA), Northern blotting, and a method utilizing DNA chips, but not always limited thereto.

The agent for measuring the level of the protein may be an antibody, a peptide, an aptamer or a compound specific for the protein.

The said antibody refers to a protein molecule capable of specifically binding to an antigenic site on a protein or peptide molecule. Such antibodies can be produced by cloning each gene into an expression vector according to a conventional method to obtain a protein encoded by the marker gene, and by a conventional method from the obtained protein. The form of the above antibody is not particularly limited, and a polyclonal antibody, a monoclonal antibody, or a part thereof having antigen binding property may be included in the antibody of the present invention, and any immunoglobulin antibody may be included. In addition, the antibody of the present invention may include a special antibody such as a humanized antibody. Furthermore, the antibody comprises a functional fragment of an antibody molecule as well as a complete form having two full-length light chains and two full-length heavy chains. The functional fragment of an antibody molecule means a fragment that possesses at least an antigen-binding function, and can be Fab, F(ab'), F(ab') 2, and Fv, etc.

The above peptide has the advantage of high binding to the target substance and does not undergo denaturation even when treated with heat or chemicals. In addition, since the above peptide has a small molecular size, it can be attached to other protein and used as a fusion protein. Specifically, it can be attached to a polymer protein chain, which can be used as a diagnostic kit and a drug delivery material.

The above aptamer refers to a single-stranded oligonucleotide, which is a nucleic acid molecule having binding activity for a specific target molecule. The above aptamer can have various three-dimensional structures depending on its nucleotide sequence and can have a high affinity for a specific substance, such as an antigen-antibody reaction. Aptamers can inhibit the activity of a specific target molecule by binding to the target molecule. Aptamers can be used as a replacement for antibodies because they are composed of polynucleotides that can specifically bind to antigenic substances in the same way as antibodies, but are more stable than proteins, have a simpler structure, and are easy to synthesize.

The method for measuring the protein level includes Western blot, enzyme linked immunosorbent assay (ELISA), radioimmunoassay (RIA), radioimmunodiffusion, ouchterlony immunodiffusion, rocket immunoelectrophoresis, tissue immunostaining, immunoprecipitation assay, complement fixation assay, FACS, and a method utilizing DNA chips, but not always limited thereto.

The present invention also provides a kit for predicting the prognosis of a colorectal cancer patient, comprising a composition containing an agent for measuring the level of mRNA of HSPD1 gene or HSP60 protein encoded by these genes.

The above kit may include an RT-PCR kit, a DNA chip kit, an ELISA kit, a protein chip kit, a rapid kit, or an MRM (multiple reaction monitoring) kit, but not always limited thereto.

The present invention also provides a method for predicting the prognosis of a colorectal cancer patient comprising the following steps:
(a) a step of measuring the level of mRNA of HSPD1 gene or HSP60 protein encoded by these genes in a sample isolated from a colorectal cancer patient; and
(b) a step of comparing the measured mRNA level or protein level thereof with the mRNA level or protein level thereof of a control sample.

If the expression level of mRNA of HSPD1 gene or HSP60 protein encoded by these genes is higher than that of the control group, it can be judged that the prognosis will be good.

The above judgment of a good prognosis may mean that the overall survival, relapse free survival, event free survival, and disease specific survival are higher than in the group with a poor prognosis.

The sample of the above step (a) may be colon tissue.

In addition, the present invention provides a method for predicting the prognosis of a colorectal cancer patient comprising the following steps:
(a) a step of measuring the level of mRNA of HSPD1 gene or HSP60 protein encoded by these genes in a sample isolated from a colorectal cancer patient;
(b) a step of comparing the measured mRNA level or protein level thereof with the mRNA level or protein level thereof of a control sample; and
(c) a step of combining and analyzing the information classified according to the TNM stage.

The TNM stage is one way to determine the stage of a tumor. T stands for tumor, and depending on the depth of invasion into the organ wall, it is divided into T0 (no primary tumor), Tis (carcinoma in situ), and T1 to T4 (the higher the number, the more invasion around it), etc. N stands for node (lymph node), and depending on the number, size, and location of the invaded lymph nodes, it is divided into N0 (no lymph node metastasis), and N1 to N3, etc. M stands for metastasis, and depending on the presence or absence of remote metastasis, it is divided into M0 (no metastasis), and M1 (metastasized), etc. Once T, N, and M are determined using the above methods they are combined to determine the final stage of a disease. The stage determined in this way is very important in determining treatment plan and prognosis.

The information categorized according to the TNM stage above is the most common way to provide prognostic information for cancer and can be divided into stages 1 through 4. However, there is a problem that predicting the prognosis of an individual patient is incomplete because the TNM stage does not include all the important variables that affect the prognosis in order to simplify the stage setting.

In the event free survival analysis of the present invention, events were defined as deaths from cancer progression, cancer recurrence, and colorectal cancer.

In the disease specific survival analysis of the present invention, deaths due to colorectal cancer were categorized as death (event), and censored deaths due to causes other than colorectal cancer were categorized as survival.

Hereinafter, the present invention will be described in detail by the following experimental examples.

However, the following experimental examples are only for illustrating the present invention, and the contents of the present invention are not limited thereto.

### Experimental Example 1: Survival prediction of colorectal cancer patients using HSPD1 gene

### <1-1> Confirmation of correlation between colorectal cancer and HSPD1 gene expression

To confirm the correlation between colorectal cancer and HSPD1 gene expression, an analysis using a genomic database was performed.

Specifically, for the survival analysis of HSP60 expression in colorectal cancer patients, we downloaded the gene expression dataset and clinical information dataset from the colon adenocarcinoma (COAD) dataset of The Cancer Genome Atlas (hereafter referred to as TCGA) project from the UCSC Cancer Genomics Browser1 (last accessed December 3, 2017). Survival analysis was performed on 272 samples with 5-year overall survival (OS) and relapse free survival (RFS) data among the primary tumor samples with expression of HSPD1, the coding gene for HSP60.

The optimal cut point for the gene expression level of HSPD1 was determined using the MaxStat function2 of R software. For overall survival (OS) analysis, patients were categorized into the HSPD1(HSP60)_{sign} group (HSPD1 expression level > 14.1791) or the HSPD1(HSP60)_{Low} group (HSPD1 expression level ≤ 14.1791). For relapse free survival (RFS) analysis, patients were classified into the HSPD1(HSP60)_{High} group (HSPD1 expression level > 14.1211) or the HSPD1(HSP60)_{Low} group (HSPD1 expression level ≤ 14.1211). The survival analysis according to the HSPD1 expression level was tested by the log-rank test, a univariate analysis. The results of multivariate analysis were corrected with gender and age using the Cox proportional hazard (CPH) model. The log-rank test and CPH modeling for survival analysis were performed using the survival package in R version 4.0. The hazard rate of each variable obtained from the CPH modeling was visualized using the forestmodel package and used under R version 4.0.

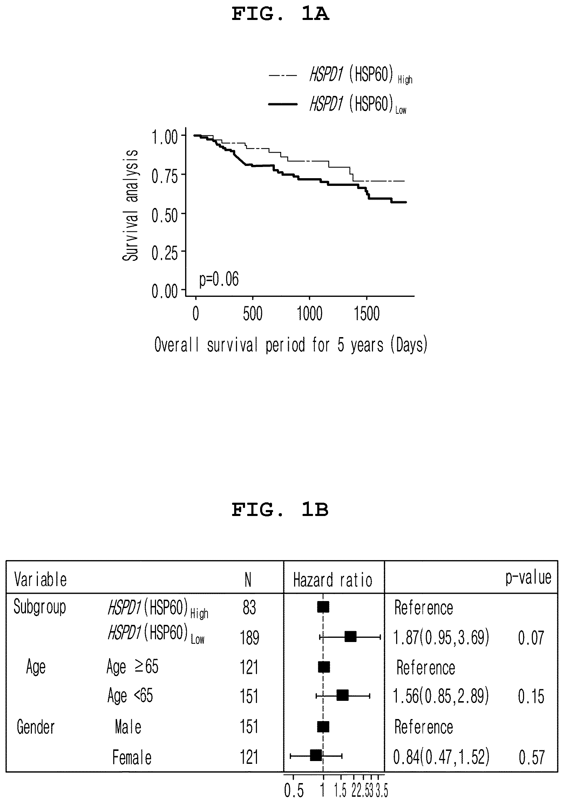

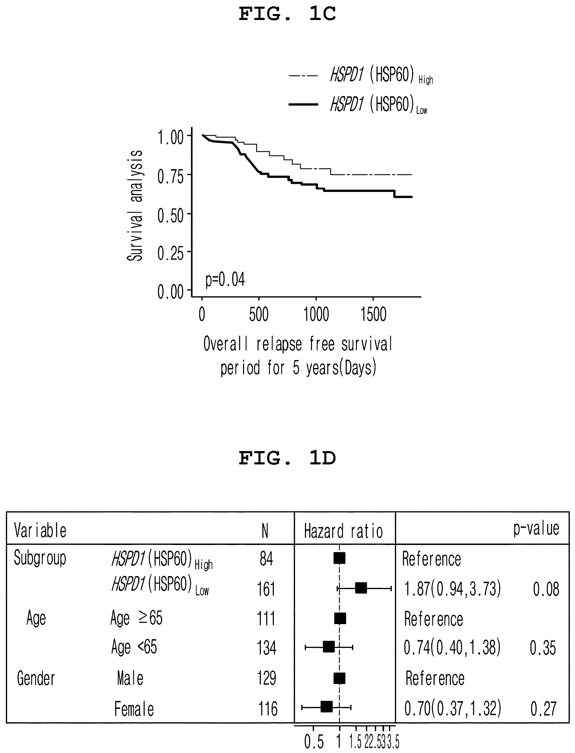

As a result, as shown in Figures 1A and 1B, the overall survival hazard ratio of the group with low expression of HSPD1 gene was 1.87 (95% confidence interval [CI]: 0.95-3.69), showing a tendency to have a worse prognosis than the group with high expression of HSPD1 gene. As shown in Figures 1C and 1D, the relapse free survival hazard ratio of the group with low expression of HSPD1 gene was 1.87 (95% confidence interval [CI]: 0.94-3.73), showing a statistically significantly worse prognosis than the group with high expression of HSPD1 gene.

The above results suggest that the patient group with high HSPD1 expression has an advantage in survival compared to the patient group with low HSPD1 expression.

### <1-2> Survival prediction of colorectal cancer patients combining TNM stage and HSPD1 gene expression level

Survival prediction was analyzed by combining the TNM stage (tumor, node, metastasis stage) and the HSPD1 gene expression level, which are accepted as the best performing biomarkers for predicting colorectal cancer patient survival.

Specifically, patients with high HSPD1 gene expression (hereinafter referred to as HSPD1(HSP60)_{High}) and TNM stage 1/2 were grouped into group 1, patients with low HSPD1 gene expression (hereinafter referred to as HSPD1 (HSP60)_{Low}) and TNM stage 1/2 were grouped into group 2, patients with HSPD1(HSP60)_{High} and TNM stage 3/4 were grouped into group 3, and patients with HSPD1(HSP60)_{Low} and TNM stage 3/4 were grouped into group 4, and 5-year overall survival and relapse free survival analyses were performed.

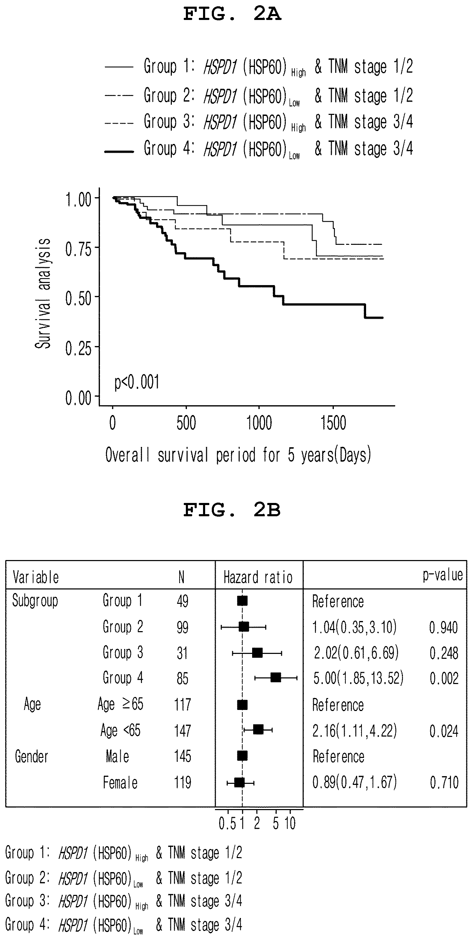

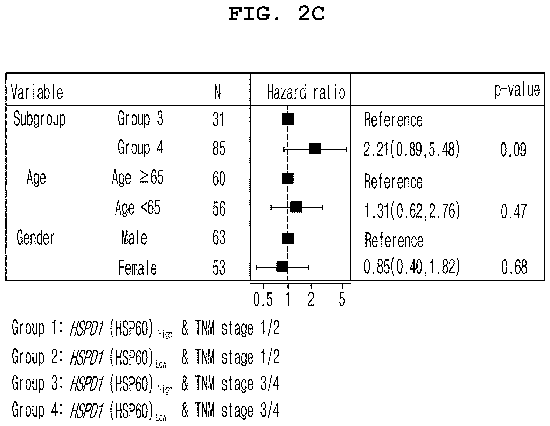

As a result, as shown in Figures 2A and 2B, when the overall survival was analyzed by combining the HSPD1 and TNM stage, the overall survival hazard ratio of group 2 was 1.04 times (95% CI: 0.35-3.10) higher than that of group 1, the overall survival hazard ratio of group 3 was 2.02 times (95% CI: 0.61-9.69), and the overall survival hazard ratio of group 4 was 5.00 times (95% CI: 1.85-13.52, log-rank test p-value: 0.002) than that of group 1. As shown in Figure 2C, in the patients with the same stage (TNM stage 3/4), the overall survival hazard ratio of the group with low HSPD1 expression (group 4) was 2.21, indicating a tendency for a worse prognosis than the group with high HSPD1 expression (group 3).

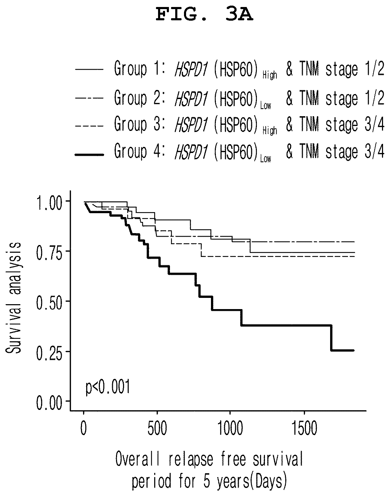

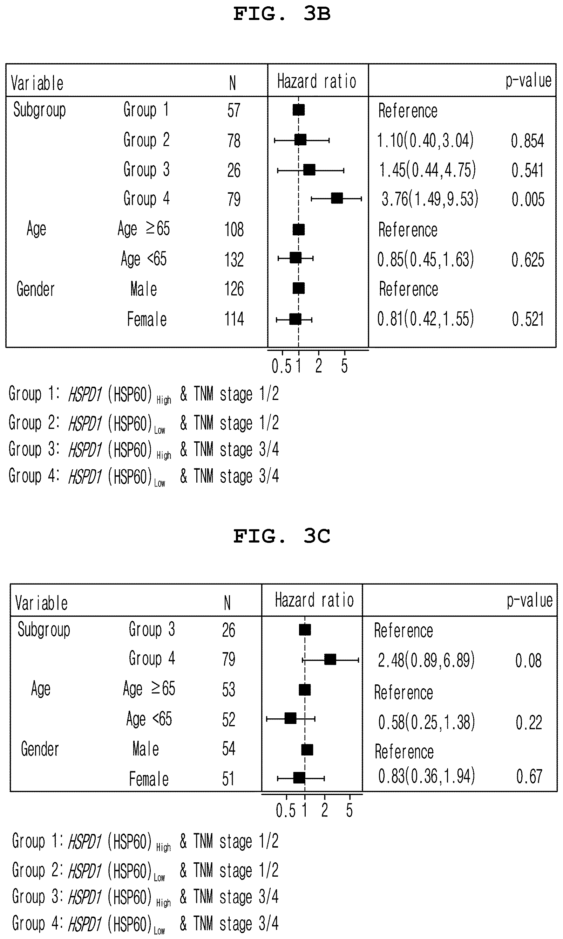

In addition, as shown in Figures 3A and 3B, when the relapse free survival was analyzed by combining the HSPD1 and TNM stage, the relapse free survival hazard ratio of group 2 was 1.10 times (95% CI: 0.40-3.04) higher than that of group 1, the relapse free survival hazard ratio of group 3 was 1.45 times (95% CI: 0.44-4.75), and the relapse free survival hazard ratio of group 4 was 3.76 times (95% CI: 1.49-9.53, log-rank test p-value: 0.005) than that of group 1. As shown in Figure 3C, in the patients with the same stage (TNM stage 3/4), the relapse free survival hazard ratio of the group with low HSPD1 expression (group 4) was 2.48, indicating a tendency for a worse prognosis than the group with high HSPD1 expression (group 3).

### Experimental Example 2: Survival prediction of colorectal cancer patients using HSP60 protein

### <2-1> Survival analysis according to HSP60 protein expression

In order to perform a survival analysis according to the expression level of the HSP60 protein encoded by the HSPD1 gene, a tissue microarray was constructed from the tumor tissues derived from colorectal cancer patients, and the expression level of the HSP60 protein was quantified by immunohistochemical staining.

Specifically, the study was conducted on patients who underwent colorectal cancer surgery at Gachon University Gil Medical Center (GMC) from April 2010 to January 2013. Patients who underwent surgery for primary colorectal cancer and patients whose tumors were preserved in paraffin blocks were included in the study, and a total of 456 patients were analyzed. Patients with recurrent colorectal cancer, patients whose normal colonic structure had been altered by previous surgery, patients who had received chemotherapy or abdominal radiation therapy prior to colorectal cancer surgery, and patients who had been treated for other cancers prior to colorectal cancer surgery were excluded. After microdissecting the paraffin blocks, Hematoxylin and Eosin (H&E) staining was performed, the pathological findings were reviewed, and two tumor cores were marked on the corresponding paraffin blocks. Cylindrical tumor tissue with a diameter of 2 mm was extracted using a tissue microarray machine and transferred to a new paraffin block. A new tissue microarray (TMA) block was created by inserting each cylindrical tissue from 69 patients into a single paraffin block. The above TMA block was cut into 4 µm thick sections using a microtome, flattened by pulling out wrinkles, and attached to a slide in a certain orientation to dry. Next, immunohistochemical staining was performed using the dried slides. The slides were treated at 60°C for 10 minutes, deparaffinized with xylene, rehydrated using different concentrations of alcohol (100% alcohol, 95% alcohol, 80% alcohol, and 70% alcohol), and then washed with distilled water. Then, for antigen recovery, 10 mM citrate buffer solution (citrate acid 2.1 g in H₂O 1 L, pH 6.0) was heated and when it started to boil, the slides were placed therein, boiled for another 10 minutes, and the slides were washed in cold water for 10 minutes, taken out, and washed in PBS buffer solution (NaCl 8 g, KCl 200 mg, Na₂HPO₄ 1.44g, KH₂PO₄ 24 mg in 1 L H₂O). Afterwards, the endogenous peroxidase activity was inhibited by treatment with 3% hydrogen peroxide for 10 minutes, and the antigen was recovered in 0.01 M sodium citrate buffer (pH 6.0) using a microwave oven. To prevent non-specific reactions, the slides were reacted with a blocking antibody (DAKO #X0909; Glostrup, Denmark) for 10 minutes at room temperature, and the samples were reacted in a humidified container at 4 °C with anti-HSP60 (rabbit monoclonal, 1:200, D6F1, Cell signaling technology, USA). The tissue slides were processed with a non-biotinylated horseradish-peroxidase (HRP) detection system according to the manufacturer's instructions (Gene Tech).

Meanwhile, statistical analysis was performed as follows. In the present invention, the survival-related factors and survival rates at 5 years after colorectal cancer surgery were the focus of event free survival and disease specific survival analyses. In the event free survival analysis, events were defined as deaths from cancer progression, cancer recurrence, and colorectal cancer. Disease specific survival was defined as the time from surgery to death due to colorectal cancer, excluding deaths due to diseases other than colorectal cancer. The log-rank test and CPH (Cox proportional hazard) modeling for survival analysis were performed using the survival package of R version 4.0. The hazard rate of each variable obtained from the CPH modeling was visualized using the forestmodel package and used under R version 4.0.

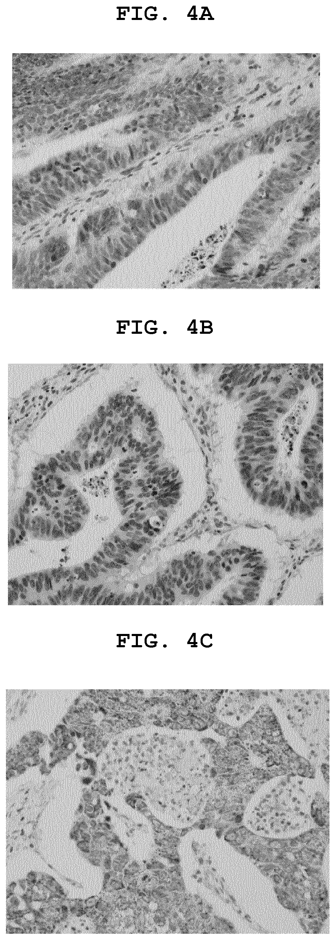

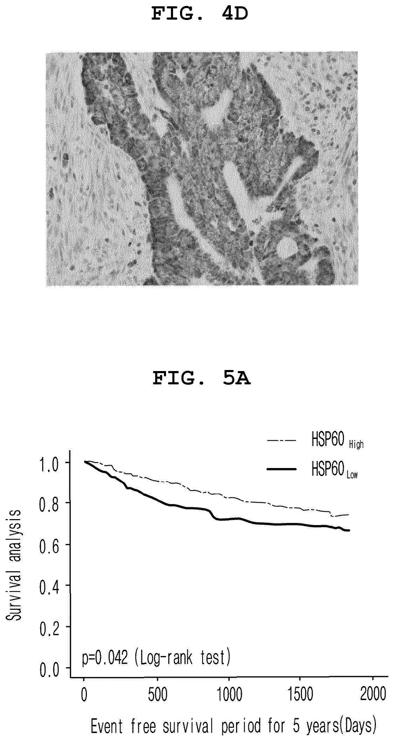

The results of immunochemical staining for HSP60 in normal colon epithelial cells and tumor cells are shown in Figure 4. Figure 4A is a photograph showing the unstained tumor cells Figure 4B is a photograph showing the tumor cells showing weak cytoplasmic staining, Figure 4C is a photograph showing the tumor cells showing moderate cytoplasmic staining, and Figure 4D is a photograph showing the tumor cells showing strong cytoplasmic staining.

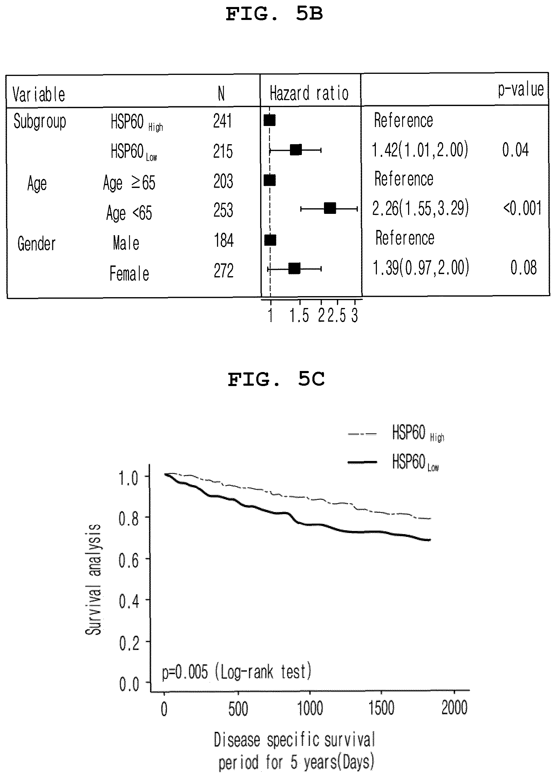

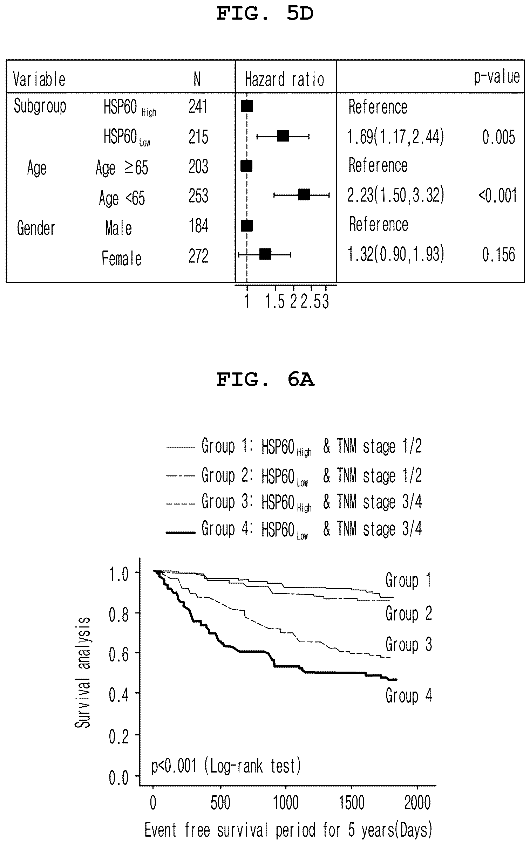

As a result of statistical analysis, as shown in Figures 5A and 5B, it was confirmed that the event free survival of colorectal cancer patients significantly differed according to the expression level of HSP60 protein. The event free survival hazard rate of the group with low expression of HSP60 protein was 1.42 times higher (95% CI: 1.01-2.00) than that of the patients with high expression of HSP60 protein, and the p-value of the CPH model corrected with gender and age was 0.04, which was statistically significant. In addition, as shown in Figures 5C and 5D, it was confirmed that the disease-specific survival of colorectal cancer patients significantly differed according to the expression level of HSP60 protein. The disease-specific survival hazard rate of the group with low expression of HSP60 protein was 1.69 times higher (95% CI: 1.17-2.44) than that of the patients with high expression of HSP60 protein, and the p-value of the CPH model corrected with gender and age was 0.005, which was statistically significant.

The above results suggest that the patient group with high HSP60 expression has an advantage in survival compared to the patient group with low HSP60 expression.

### <2-2> Survival prediction of colorectal cancer patients combining TNM stage and HSP60 protein expression level

Survival prediction was analyzed by combining the TNM stage and the HSP60 protein expression level.

Specifically, patients with high HSP60 protein expression (hereinafter referred to as HSP60_{High}) and TNM stage 1/2 were grouped into group 1, patients with low HSP60 protein expression (hereinafter referred to as HSP60_{Low}) and TNM stage 1/2 were grouped into group 2, patients with HSP60_{High} and TNM stage 3/4 were grouped into group 3, and patients with HSP60_{Low} and TNM stage 3/4 were grouped into group 4, and 5-year event free survival and disease-specific survival analyses were performed. In addition, the survival analysis was tested by the log-rank test, a univariate analysis, and the CPH modeling, a multivariate analysis. The log-rank test and the CPH modeling for survival analysis were performed using the survival package in R version 4.0. The hazard rate of each variable obtained from the CPH modeling was visualized using the forestmodel package and used under R version 4.0.

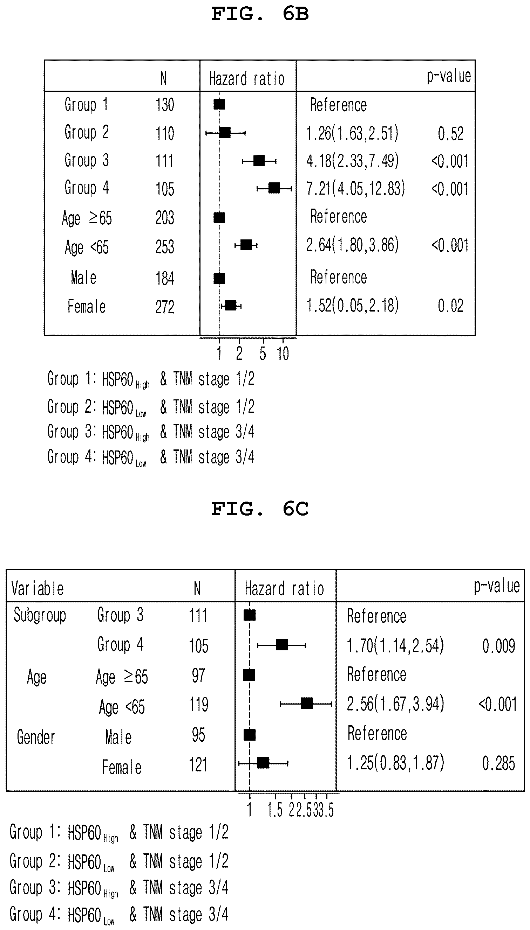

As a result, as shown in Figures 6A and 6B, when the event free survival was analyzed by combining the HSP60 and TNM stage, the event free survival hazard ratio of group 3 was 4.18 times (95% CI: 2.33-7.49, p-value: <0.001) higher than that of group 1, and the event free survival hazard ratio of group 4 was 7.21 times (95% CI: 4.05-12.83, p-value: <0.001) higher than that of group 1. As shown in Figure 6C, in the patients with the same stage (TNM stage 3/4), the event free survival hazard ratio of the group with low HSP60 expression was 1.70, indicating a tendency for a significantly worse prognosis (p-value=0.009) than the group with high HSP60 expression. This suggests that better performance in survival prediction is possible when the HSP60 expression is combined with the TNM stage. The above results suggest that combining the HSP60 expression and TNM stage can provide better survival prediction performance than predicting survival using the TNM stage alone.

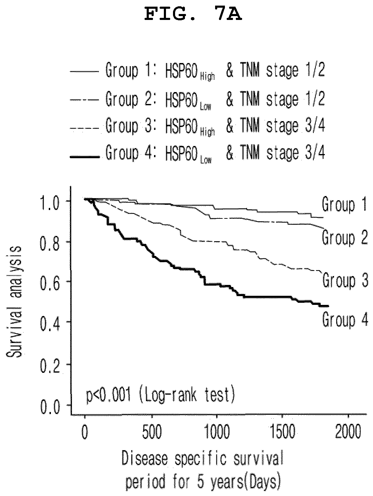

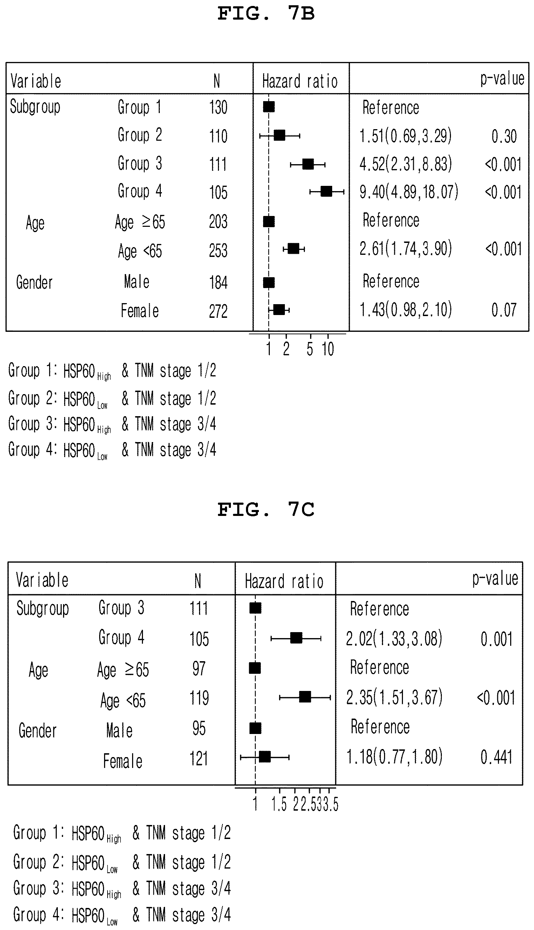

In addition, as shown in Figures 7A and 7B, when the disease-specific survival was analyzed by combining the HSP60 and TNM stage, the disease-specific survival hazard ratio of group 3 was 4.52 times (95% CI: 2.31-8.83, p-value: <0.001) higher than that of group 1, and the disease-specific survival hazard ratio of group 4 was 9.40 times (95% CI: 4.89-18.07, p-value: <0.001) higher than that of group 1. As shown in Figure 7C, in the patients with the same stage (TNM stage 3/4), the disease-specific survival hazard ratio of the group with low HSP60 expression was 2.02, indicating a tendency for a significantly worse prognosis (p-value=0.001) than the group with high HSP60 expression.

Through the above results, it was confirmed that when the HSP60 expression level and TNM stage were combined to predict survival, better survival prediction was possible than when the TNM stage was used alone to predict survival. This suggests that better performance in survival prediction is possible when the HSP60 expression level is combined with the TNM stage and used as a biomarker.

## Claims

1. A composition for predicting the prognosis of a colorectal cancer patient, comprising an agent for measuring the level of mRNA of HSPD1 gene or HSP60 protein encoded by these genes.

2. The composition for predicting the prognosis of a colorectal cancer patient according to claim 1, wherein the agent for measuring the mRNA level is a primer or probe that specifically binds to said gene.

3. The composition for predicting the prognosis of a colorectal cancer patient according to claim 1, wherein the agent for measuring the protein level includes an antibody, peptide, aptamer or compound specific for said protein.

4. A kit for predicting the prognosis of a colorectal cancer patient comprising the composition of claim 1.

5. A method for predicting the prognosis of a colorectal cancer patient comprising the following steps:
(a) a step of measuring the level of mRNA of HSPD1 gene or HSP60 protein encoded by these genes in a sample isolated from a colorectal cancer patient; and
(b) a step of comparing the measured mRNA level or protein level thereof with the mRNA level or protein level thereof of a control sample.

6. The method for predicting the prognosis of a colorectal cancer patient according to claim 5, wherein the prognosis is determined to be good if the level of mRNA of HSPD1 gene or HSP60 protein encoded by these genes is higher than that of the control group.

7. The method for predicting the prognosis of a colorectal cancer patient according to claim 5, wherein the prognosis is determined to be poor if the level of mRNA of HSPD1 gene or HSP60 protein encoded by these genes is lower than that of the control group.

8. The method for predicting the prognosis of a colorectal cancer patient according to claim 5, wherein the sample of step (a) above is the colon tissue.

9. A method for predicting the prognosis of a colorectal cancer patient comprising the following steps:
(a) a step of measuring the level of mRNA of HSPD1 gene or HSP60 protein encoded by these genes in a sample isolated from a colorectal cancer patient;
(b) a step of comparing the measured mRNA level or protein level thereof with the mRNA level or protein level thereof of a control sample; and
(c) a step of combining and analyzing the information classified according to the TNM stage (tumor, node, metastasis stage).

10. The method for predicting the prognosis of a colorectal cancer patient according to claim 9, wherein the prognosis is determined to be good if the level of mRNA of HSPD1 gene or HSP60 protein encoded by these genes is higher than that of the control group in the same TNM stage.

11. The method for predicting the prognosis of a colorectal cancer patient according to claim 9, wherein the prognosis is determined to be poor if the level of mRNA of HSPD1 gene or HSP60 protein encoded by these genes is lower than that of the control group in the same TNM stage.
